# EUROPEAN PATENT SPECIFICATION

(11) **EP 4 280 998 B1**
(45) Date of publication and mention of the grant of the patent: **02.12.2026**
(21) Application number: 22786116.8
(22) Date of filing: 02.10.2022
(51) Int. Cl.: A61B 34/30, A61B 34/00, A61B 90/20, A61B 90/00

(54) **ONE-SIDED ROBOTIC SURGICAL PROCEDURE**
EINSEITIGES ROBOTISCHES CHIRURGISCHES VERFAHREN
ACTE CHIRURGICAL ROBOTIQUE UNILATÉRAL

(30) Priority: 17.10.2021 US 202163256587 P
(43) Date of publication of application: 29.11.2023
(73) Proprietor: Forsight Robotics Ltd., Caesarea 3079566 (IL)
(72) Inventor: VAKNIN, Oshri, 6451802 Tel Aviv (IL); GLOZMAN, Daniel, 4034723 Kfar Yona (IL)
(74) Representative: Evens, Paul Jonathan
(86) International application number: PCT/IB2022/059386
(87) International publication number: WO 2023/062470

(56) References cited:
- US-A1- 2010 010 504
- US-A1- 2016 270 867
- US-A1- 2020 397 520
- US-A1- 2020 397 531

## Description

### CROSS-REFERENCES TO RELATED APPLICATIONS

The present application claims priority from U.S. Provisional Patent Application No. 63/256,587 to Vaknin, filed October 17, 2021, entitled "One-sided robotic surgical procedure".

### FIELD OF EMBODIMENTS OF THE DISCLOSURE

Some applications of the present disclosure generally relate to medical apparatus and methods. Specifically, some applications of the present disclosure relate to apparatus and methods for performing microsurgical procedures in a robotic manner.

### BACKGROUND

Cataract surgery involves the removal of the natural lens of the eye that has developed an opacification (known as a cataract), and its replacement with an intraocular lens. Such surgery typically involves a number of standard steps, which are performed sequentially.

In an initial step, the patient's face around the eye is disinfected (typically, with iodine solution), and their face is covered by a sterile drape, such that only the eye is exposed. When the disinfection and draping has been completed, the eye is anesthetized, typically using a local anesthetic, which is administered in the form of liquid eye drops. The eyeball is then exposed, using an eyelid speculum that holds the upper and lower eyelids open. One or more incisions (and typically two or three incisions) are made in the cornea of the eye. The incision(s) are typically made using a specialized blade, that is called a keratome blade. At this stage, lidocaine is typically injected into the anterior chamber of the eye, in order to further anesthetize the eye. Following this step, a viscoelastic injection is applied via the corneal incision(s). The viscoelastic injection is performed in order to stabilize the anterior chamber and to help maintain eye pressure during the remainder of the procedure, and also in order to distance the anterior lens capsule from the cornea.

In a subsequent stage, known as capsulorhexis, a part of the anterior lens capsule is removed. Various enhanced techniques have been developed for performing capsulorhexis, such as laser-assisted capsulorhexis, zepto-rhexis (which utilizes precision nano-pulse technology), and marker-assisted capsulorhexis (in which the cornea is marked using a predefined marker, in order to indicate the desired size for the capsule opening).

Subsequently, it is common for a fluid wave to be injected via the corneal incision, in order to dissect the cataract's outer cortical layer, in a step known as hydrodissection. In a subsequent step, known as hydrodelineation, the outer softer epi-nucleus of the lens is separated from the inner firmer endo-nucleus by the injection of a fluid wave. In the next step, ultrasonic emulsification of the lens is performed, in a process known as phacoemulsification. The nucleus of the lens is broken initially using a chopper, following which the outer fragments of the lens are broken and removed, typically using an ultrasonic phacoemulsification probe. Further typically, a separate tool is used to perform suction during the phacoemulsification. When the phacoemulsification is complete, the remaining lens cortex (i.e., the outer layer of the lens) material is aspirated from the capsule. During the phacoemulsification and the aspiration, aspirated fluids are typically replaced with irrigation of a balanced salt solution, in order to maintain fluid pressure in the anterior chamber. In some cases, if deemed to be necessary, then the capsule is polished. Subsequently, the intraocular lens (IOL) is inserted into the capsule. The IOL is typically foldable and is inserted in a folded configuration, before unfolding inside the capsule. At this stage, the viscoelastic is removed, typically using the suction device that was previously used to aspirate fluids from the capsule. If necessary, the incision(s) is sealed by injecting balanced salt solution into the corneal stroma until diffuse whitening occurs, thereby causing the internal tissue to be pressed against the external tissue of the incision, such as to force closed the incision.

US 2016/270867 A1 describes a robotic system with a camera, a display and two robotic arms, each arm controlled by a respective input device. The controller has a mirror mode, mirroring the image on the display and the outputs of the robotic unit, if the operator prefers to operate mainly with the dominant hand.

US 2010/010504 A1 describes a robotic system for an ophthalmic procedure with a surgical microscope, and two robotic arms, each arm controlled by a respective input device.

### SUMMARY OF THE INVENTION

The invention is defined by the appended independent claims. Further embodiments of the invention are illustrated in the dependent claims. The methods described herein do not form part of the invention, but are useful for understanding the invention.

### SUMMARY OF THE DISCLOSURE

In accordance with some applications of the present disclosure, apparatus and methods are provided that allow a surgeon to perform a procedure on a portion of a first side of patient's body, as if (s)he is performing the procedure on the same portion but on the opposite side of the patient's body. In this manner, if the surgeon has a preferred side of the body upon which to perform the procedure, even when (s)he is performing the procedure on the less-preferred side they can perform the procedure as if they were performing the procedure on their preferred side. For example, cataract surgery, as well as other ophthalmic surgeries, are typically performed upon both the patient's eyes (usually in two separate procedures). It follows that, when performing surgery on the patient's left eye, the surgeon's hands will be positioned differently with respect to the patient's face relative to when the surgeon is performing surgery on the patient's right eye. As most surgeons are not ambidextrous, surgeons report discomfort and reachability issues while positioning their dominant hand on the patient's nasal and/or medial frontal bones for stability. This discomfort occurs in cataract surgery where the surgeon sits superior to the patient's head, and the eye being operated on is in the opposed position to the surgeon's dominant hand. This is in contrast to when surgeons position their dominant hand on the patient's lateral frontal bone (bordering with the zygomatic arch bone), when performing surgery on the opposite eye. Thus, it is typically the case that right-handed surgeons have a preference for ophthalmic procedures that are performed on patients' left eye, whereas left-handed surgeons prefer to perform ophthalmic procedures on patients' right eyes.

For some applications, a robotic system includes one or more robotic units (which are configured to hold tools). Typically, the robotic system includes one or more control components (e.g., joysticks), each of which is configured to control actions of a respective one of the robotic units. Typically, when robotic system is used to perform surgery on the side of the body upon which the surgeon feels more comfortable operating, a display displays a non-mirrored image of the portion of the body to the surgeon and the surgeon uses each of the control components to control the respective corresponding robotic unit. Typically, when the robotic system is used to perform surgery on the side of the body upon which the surgeon feels less comfortable operating, the display displays a mirror image of the portion of the body to the surgeon and the surgeon uses the each of the control components to control the respective mirror robotic unit with the inputs to the control components being mirrored by the robotic units.

For example, as described hereinabove, an ophthalmic surgeon who is right handed typically prefers to perform ophthalmic procedures on patients' left eyes. In such cases, in order to perform a procedure on the patient's left eye, the system will typically not utilize the mirror functionalities described herein, since the surgeon is comfortable performing the procedure without the image and the control components having the mirroring functionalities. However, when performing a procedure on the patient's right eye, the mirroring functionalities described herein are typically implemented, such that even when the surgeon performs surgery on the patient's right eye it is as if (s)he is performing the surgery on their left eye.

There is therefore provided, in accordance with some applications of the present disclosure, an apparatus for performing a procedure on a portion of a first side of a body of a patient using one or more tools, the apparatus including:
an imaging device configured to image the portion of the first side of the patient's body;
a display configured to display a mirror image of the portion of the first side of the patient's body to an operator;
one or more control components configured to be operated by the operator;
one or more robotic units configured to hold the one or more tools, each of the one or more robotic units corresponding to a respective control component and mirroring a position of the corresponding control component; and
at least one computer processor configured to process inputs into the each of the control components such as to generate mirrored outputs at the corresponding robotic unit.

In some applications, the display is configured to display a mirror image of the portion of the first side of the patient's body to the operator, such that the mirror image appears to the operator as if it is the opposite side of the patient's body to the first side of the patient's body.

In some applications, the operator is configured to move the control components with respect to the mirror image, such that it is as if the operator is virtually operating on the opposite side of the patient's body to the first side of the patient's body.

In some applications, the one or more control components include one or more joysticks.

In some applications, the one or more joysticks are configured to hold virtual tools that corresponds to the one or more tools.

In some applications, the computer processor is configured to cause a tip of the one of the one or more tools to move such that it mirrors movement of a tip of a corresponding one of the one or more virtual tools.

In some applications:
the one or more control components include a first control component configured to be operated by a left hand of the operator and a second control component configured to be operated by a right hand of the operator; and
the one or more robotic units include a first robotic unit configured to hold a first one of the one or more tools, and a second robotic unit being configured to hold a second one of the one or more tools, the first robotic unit corresponding to the first control component and mirroring a position of the first control component, and the second robotic unit corresponding to the second control component and mirroring a position of the second control component.

In some applications, the first and second control components are configured to be placed at locations with respect to the mirror image of the portion of the first side of the patient's body that corresponds to locations of the first and second robotic units with respect to the portion of the first side of the patient's body.

In some applications, he first robotic unit is configured to hold the first tool with respect to the portion of the first side of the patient's body at a location that is disposed to the right of a location at which the second robotic unit is configured to hold the first tool with respect to the portion of the second side of the patient's body, when viewed in a direction facing the first side of the patient's body from a location on the first side of the patient's body, and the computer processor is configured to process inputs into the first control component such as to generate a mirrored output at the first robotic unit, and to process inputs into the second control component such as to generate a mirrored output at the second robotic unit.

In some applications, the apparatus is configured for use with an eye on a first side of the patient's body, the imaging device is configured to image the eye on the first side of the patient's body and the display is configured to display a mirror image of the eye on the first side of the patient's body, such that the mirror image appears to the operator as if it is the opposite eye to the eye on the first side of the patient's body.

In some applications, the operator is configured to move the control components with respect to the mirror image, such that it is as if the operator is virtually operating on the opposite eye to the eye on the first side of the patient's body.

In some applications, the first and second control components are configured to be placed at locations with respect to the mirror image of the eye of the first side of the patient's body that corresponds to locations of the first and second robotic units with respect to the eye of the first side of the patient's body.

In some applications, the first robotic unit is configured to hold the first tool with respect to the eye of the first side of the patient's body at a location that is disposed to the right of a location at which the second robotic unit is configured to hold the second tool with respect to the eye of the first side of the patient's body, when viewed in a direction facing the eye on the first side of the patient's body from a location on the first side of the patient's body, and the computer processor is configured to process inputs into the first control component such as to generate a mirrored output at the first robotic unit, and to process inputs into the second control component such as to generate a mirrored output at the second robotic unit.

There is further provided, in accordance with some applications of the present disclosure, a method for performing a procedure on a portion of a first side of a body of a patient using one or more tools that are held by one or more robotic units, the method including:
driving an imaging device to image the portion of the first side of the patient's body;
driving a display to display a mirror image of the portion of the first side of the patient's body to an operator;
using a computer processor:
   receiving inputs via one or more control components configured to be operated by the operator, each of the one or more robotic units corresponds to a respective control component and mirrors a position of the corresponding control component; and
   processing the inputs into the each of the control components such as to generate mirrored outputs at the corresponding robotic unit.

In some applications, driving the display to display a mirror image of the portion of the first side of the patient's body to the operator includes driving the display to display a mirror image of the portion of the first side of the patient's body to the operator, such that the mirror image appears to the operator as if it is the opposite side of the patient's body to the first side of the patient's body.

In some applications, receiving inputs via one or more control components configured to be operated by the operator includes allowing the operator to move the control components with respect to the mirror image, such that it is as if the operator is virtually operating on the opposite side of the patient's body to the first side of the patient's body.

In some applications, receiving inputs via one or more control components configured to be operated by the operator includes receiving inputs via one or more joysticks configured to be operated by the operator.

In some applications, receiving inputs via the one or more joysticks configured to be operated by the operator includes receiving inputs via one or more joysticks that are configured to hold virtual tools that corresponds to the one or more tools.

In some applications, processing the inputs into the each of the control components such as to generate mirrored outputs at the corresponding robotic unit includes causing a tip of the one of the one or more tools to move such that it mirrors movement of a tip of a corresponding one of the one or more virtual tools.

In some applications:
a first robotic unit is configured to hold a first one of the one or more tools, and a second robotic unit is configured to hold a second one of the one or more tools;
receiving inputs via one or more control components configured to be operated by the operator includes receiving inputs via a first control component configured to be operated by a left hand of the operator and a second control component configured to be operated by a right hand of the operator; and
processing the inputs into the each of the control components such as to generate mirrored outputs at the corresponding robotic unit includes moving the first robotic unit such as to mirror a position of the first control component, and moving the second robotic unit such as to correspond to mirror a position of the second control component.

In some applications, the first and second control components are configured to be placed at locations with respect to the mirror image of the portion of the first side of the patient's body that corresponds to locations of the first and second robotic units with respect to the portion of the first side of the patient's body.

In some applications, the first robotic unit is configured to hold the first tool with respect to the portion of the first side of the patient's body at a location that is disposed to the right of a location at which the second robotic unit is configured to hold the first tool with respect to the portion of the second side of the patient's body, when viewed in a direction facing the first side of the patient's body from a location on the first side of the patient's body, and processing the inputs into the each of the control components such as to generate mirrored outputs at the corresponding robotic unit includes processing inputs into the first control component such as to generate a mirrored output at the first robotic unit, and processing inputs into the second control component such as to generate a mirrored output at the second robotic unit.

In some applications:
the method is configured for use with an eye on a first side of the patient's body;
driving the imaging device to image the portion of the first side of the patient's body includes driving the imaging device to image the eye on the first side of the patient's body; and
driving the display to display the mirror image of the portion of the first side of the patient's body to an operator includes driving the display to display a mirror image of the eye on the first side of the patient's body, such that the mirror image appears to the operator as if it is the opposite eye to the eye on the first side of the patient's body.

In some applications, receiving inputs via one or more control components configured to be operated by the operator includes allowing the operator to move the control components with respect to the mirror image, such that it is as if the operator is virtually operating on the opposite eye to the eye on the first side of the patient's body.

In some applications, the first and second control components are configured to be placed at locations with respect to the mirror image of the eye of the first side of the patient's body that corresponds to locations of the first and second robotic units with respect to the eye of the first side of the patient's body.

In some applications, the first robotic unit is configured to hold the first tool with respect to the eye of the first side of the patient's body at a location that is disposed to the right of a location at which the second robotic unit is configured to hold the second tool with respect to the eye of the first side of the patient's body, when viewed in a direction facing the eye on the first side of the patient's body from a location on the first side of the patient's body, and processing the inputs into the each of the control components such as to generate mirrored outputs at the corresponding robotic unit includes processing inputs into the first control component such as to generate a mirrored output at the first robotic unit, and processing inputs into the second control component such as to generate a mirrored output at the second robotic unit.

The present disclosure will be more fully understood from the following detailed description of applications thereof, taken together with the drawings, in which:

### BRIEF DESCRIPTION OF THE DRAWINGS

Fig. 1 is a schematic illustration of a robotic system that is configured for use in a microsurgical procedure, such as intraocular surgery, in accordance with some applications of the present disclosure;
Fig. 2 is a schematic illustration of a surgeon performing ophthalmic surgery on a patient in a traditional manner (i.e., using non-robotic surgery), in accordance with prior art techniques; and
Figs. 3A and 3B are respectively an original image and a mirror image of an eye of a subject, in accordance with some applications of the present disclosure.

### DETAILED DESCRIPTION OF EMBODIMENTS

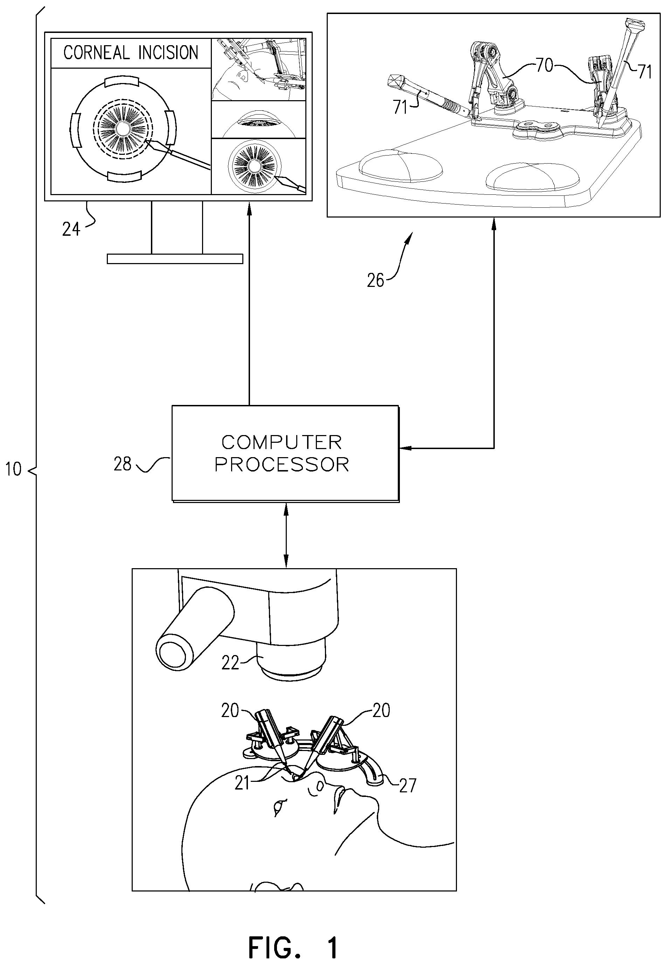

Reference is now made to Fig. 1, which is a schematic illustration of a robotic system 10 that is configured for use in a microsurgical procedure, such as intraocular surgery, in accordance with some applications of the present disclosure.

Typically, when used for intraocular surgery, robotic system 10 includes one or more robotic units 20 (which are configured to hold tools 21), in addition to an imaging system 22, a display 24 and a control component unit 26 (e.g., a pair of control components, such as joysticks, as shown), via which a user (e.g., a healthcare professional) is able to control robotic units 20. Typically, robotic system 10 includes one or more computer processors 28, via which components of the system and a user (e.g., a healthcare professional) operatively interact with each other. For some applications, each of the robotic units is supported on a base 27, as shown. The scope of the present application includes mounting one or more robotic units in any of a variety of different positions with respect to each other.

Typically, movement of the robotic units (and/or control of other aspects of the robotic system) is at least partially controlled by a user (e.g., a healthcare professional). For example, the user may receive images of the patient's eye and the robotic units, and/or tools disposed therein, via display 24. Typically, such images are acquired by imaging system 22. For some applications, imaging system 22 is a stereoscopic imaging device and display 24 is a stereoscopic display. Based on the received images, the user typically performs steps of the procedure. For some applications, the user provides commands to the robotic units via control component unit 26. Typically, such commands include commands that control the position and/or orientation of tools that are disposed within the robotic units, and/or commands that control actions that are performed by the tools. For example, the commands may control a blade, a phacoemulsification tool (e.g., the operation mode and/or suction power of the phacoemulsification tool), and/or injector tools (e.g., which fluid (e.g., viscoelastic fluid, saline, etc.) should be injected, and/or at what flow rate). Alternatively or additionally, the user may input commands that control the imaging system (e.g., the zoom, focus, and/or x-y positioning of the imaging system). For some applications, the commands include controlling an IOL-manipulator tool, for example, such that the tool manipulates the IOL inside the eye for precise positioning of the IOL within the eye.

Typically, control component unit 26 includes one or more control components 70, e.g., joysticks that are configured to correspond to respective robotic units 20 of the robotic system. For example, as shown, the system may include first and second robotic units, and the control component unit may include first and second control components, e.g., joysticks, as shown. Typically, each of the joysticks is a control-component arm that includes a plurality of links that are coupled to each other via joints. For some applications, the control-component joysticks are configured to hold respective control-component tools 71 therein (in order to replicate the arms of the robotic units), as shown in Fig. 1A. Typically, the computer processor determines the XYZ location and orientation of the tip of the control-component tool 71, and drives the robotic unit such that the tip of the actual tool that is being used to perform the procedure tracks the movements of the tip of the control-component tool. As described in further detail hereinbelow, in some cases, the movement of the tip of the actual tool that is used to perform the procedure is made to mirror the movements of the tip of the control-component tool.

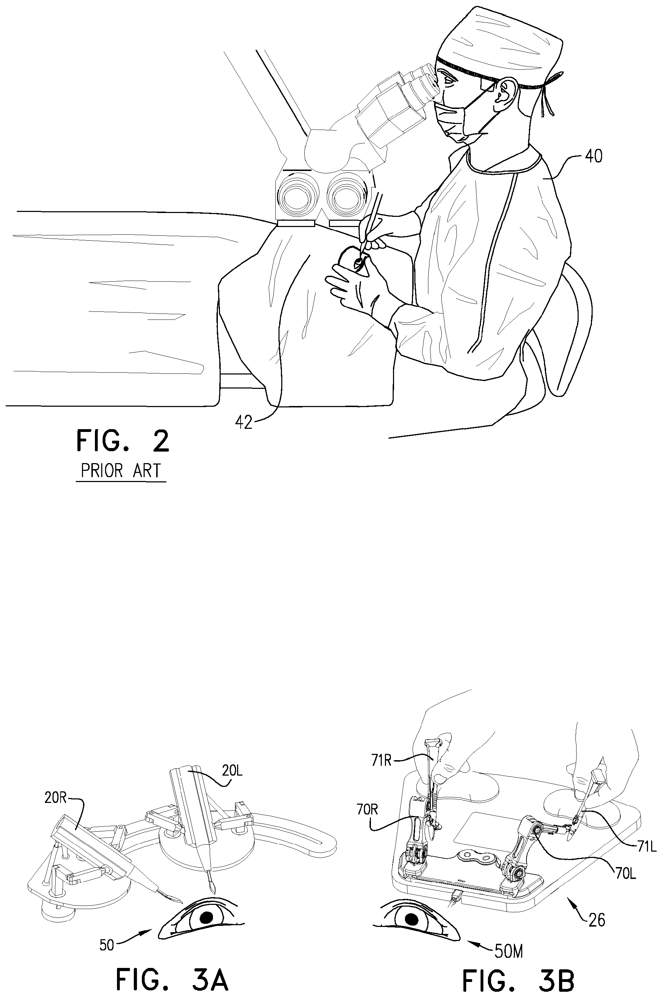

Reference is also made to Fig. 2, which is a schematic illustration of a surgeon 40 performing ophthalmic surgery on a patient 42 in a traditional manner (i.e., using non-robotic surgery), in accordance with prior art techniques. Cataract surgery, as well as other ophthalmic surgeries, are typically performed upon both the patient's eyes (usually in two separate procedures). Further typically, when performing such surgery in a traditional manner (i.e., using non-robotic surgery), various stages of the procedure are performed by the surgeon operating two tools simultaneously, with each tool being manipulated using a respective hand. It follows that, when performing surgery on the patient's left eye, the surgeon's hands will be positioned differently with respect to the patient's face relative to when the surgeon is performing surgery on the patient's right eye. As most surgeons are not ambidextrous, surgeons report discomfort and reachability issues while positioning their dominant hand on the patient's nasal and/or medial frontal bones for stability. This discomfort occurs in cataract surgery where the surgeon sits superior to the patient's head, and the eye being operated on is in the opposed position to the surgeon's dominant hand. This is in contrast to when surgeons position their dominant hand on the patient's lateral frontal bone (bordering with the zygomatic arch bone), when performing surgery on the opposite eye. Thus, it is typically the case that right-handed surgeons have a preference for ophthalmic procedures that are performed on patients' left eye, whereas left-handed surgeons prefer to perform ophthalmic procedures on patients' right eyes. It is noted that the above-described discomfort may even extend to when surgeons perform microsurgical procedures in which they do not actually rest position their hands on patients' faces. This may result from surgeons "learning" to prefer performing a procedure on one side of patients' faces or the other, due to their acquired experiences of the above-mentioned issues of discomfort and reachability.

Referring again to Fig. 1, for some applications, robotic system 10 includes first and second robotic units 20 (which are configured to hold tools 21). Typically, each of the robotic units is configured to perform actions that are performed by a respective one of the surgeon's hands. Further typically, the robotic system includes control component unit 26, which typically includes a pair of control components 70, e.g. joysticks, with each of the control components typically being configured to control actions of a respective one of the robotic units. Typically, each of the joysticks is a control-component arm that includes a plurality of links that are coupled to each other via joints. Typically, when robotic system is used to perform surgery on the eye upon which the surgeon feels more comfortable operating, display 24 displays a non-mirrored image of the eye to the surgeon and the surgeon uses each of the control components to control the respective corresponding robotic arm. For some applications of the present disclosure, when the robotic system is used to perform surgery on the eye upon which the surgeon feels less comfortable operating, display 24 displays a mirror image of the eye to the surgeon and the surgeon uses the each of the control components to control the respective mirror robotic unit with the inputs to the control components being mirrored by the robotic units. It is noted that this is possible because (a) the eye is an asymmetric organ, (b) display 24 is configured to display a mirror image of the patient's eye, and (c) computer processor 28 is configured to process inputs into the control components such as to generate mirrored outputs at the robotic units.

Referring now to Figs. 3A and 3B, an example of the above-described procedure is provided. Fig. 3A is a non-mirror image 50 of a patient's right eye. Fig. 3B shows a mirror image 50M of the right eye (which appears similar to a left eye). The example that is provided illustrates a case in which a surgeon (or another operator of robotic system 10) prefers to perform a procedure on patients' left eyes, which is typically the case for a surgeon who is right handed. In such cases, in order to perform a procedure on the patient's left eye, the system will typically not utilize the mirror functionalities described herein, since the surgeon is comfortable performing the procedure without the image and the control components having the mirroring functionalities. However, when performing a procedure on the patient's right eye (shown in Fig. 3A), the mirroring functionalities described herein are typically implemented, such that even when the surgeon performs surgery on the patient's right eye it is as if (s)he is performing the surgery on their left eye.

Still referring to Figs. 3A and 3B, in the above-described scenario, in order to perform a procedure on the patient's right eye, display 24 is configured to display mirror image 50M of the right eye to the surgeon, such that the surgeon sees what appears to be a left eye (as shown in Fig. 3B). The surgeon then positions and manipulates control components of 70 control component unit 26 (e.g., joysticks) as if (s)he was performing surgery on the patient's left eye, while robotic units 20 are positioned in corresponding, mirror positions with respect to the patient's right eye. When the surgeon moves and/or provides another input to one of the control components, the computer processor is configured to direct the input to the corresponding, mirror robotic unit 20 and to process the input such as to generate a mirrored output at the robotic unit. Thus, when the surgeon manipulates a control component 70L that is controlled by her/his left hand (e.g., a left joystick), it generates a mirrored output at the robotic unit that is positioned with respect to the patient's right eye at a position that is such that its movement would normally be controlled by a surgeons' right hand (denoted by 20R). Similarly, when the surgeon manipulates a control component 70R that is controlled by her/his right hand (e.g., a right joystick), it generates a mirrored output at the robotic unit that is positioned with respect to the patient's right eye at a position that is such that its movement would normally be controlled by a surgeons' left hand (denoted by 20L). In this manner, the surgeon is able to operate on both of the patient's eyes, while artificially viewing only her/his preferred eye and manipulating the control components as if to operate on his/her preferred eye.

It is noted that although some applications of the disclosure are described with reference to a robotic system that includes two robotic units and a control component unit that includes two control components (e.g., joysticks), which correspond to respective robotic units, the scope of the present disclosure includes a robotic system that includes any number of robotic units and a corresponding number of control components (e.g., joysticks or an alternative type of control components). For example, some applications of the present disclosure are applicable to a robotic system that includes a single robotic unit and a control component unit that includes a single control component (e.g., joystick or an alternative type of control component). Alternatively, some applications of the present disclosure are applicable to a robotic system that includes more than two robotic units and a control component unit that includes a corresponding number of control components (e.g., joysticks or an alternative type of control components).

Although some applications of the present disclosure are described with reference to cataract surgery, the scope of the present application includes applying the apparatus and methods described herein to other medical procedures, *mutatis mutandis.* In particular, the apparatus and methods described herein to other medical procedures may be applied to other microsurgical procedures, such as general surgery, orthopedic surgery, gynecological surgery, otolaryngology, neurosurgery, oral and maxillofacial surgery, plastic surgery, podiatric surgery, vascular surgery, and/or pediatric surgery that is performed using microsurgical techniques. For some such applications, the imaging system includes one or more microscopic imaging units.

It is noted that the scope of the present application includes applying the apparatus and methods described herein to intraocular procedures, other than cataract surgery, *mutatis mutandis.* Such procedures may include collagen crosslinking, endothelial keratoplasty (e.g., DSEK, DMEK, and/or PDEK), DSO (descemets stripping without transplantation), laser assisted keratoplasty, keratoplasty, LASIK/PRK, SMILE, pterygium, ocular surface cancer treatment, secondary IOL placement (sutured, transconjunctival, etc.), iris repair, IOL reposition, IOL exchange, superficial keratectomy, Minimally Invasive Glaucoma Surgery (MIGS), limbal stem cell transplantation, astigmatic keratotomy, Limbal Relaxing Incisions (LRI), amniotic membrane transplantation (AMT), glaucoma surgery (e.g., trabs, tubes, minimally invasive glaucoma surgery), automated lamellar keratoplasty (ALK), anterior vitrectomy, and/or pars plana anterior vitrectomy.

Applications of the disclosure described herein can take the form of a computer program product accessible from a computer-usable or computer-readable medium (e.g., a non-transitory computer-readable medium) providing program code for use by or in connection with a computer or any instruction execution system, such as computer processor 28. For the purpose of this description, a computer-usable or computer readable medium can be any apparatus that can comprise, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Typically, the computer-usable or computer readable medium is a non-transitory computer-usable or computer readable medium.

Examples of a computer-readable medium include a semiconductor or solid-state memory, magnetic tape, a removable computer diskette, a random-access memory (RAM), a read-only memory (ROM), a rigid magnetic disk and an optical disk. Current examples of optical disks include compact disk-read only memory (CD-ROM), compact disk-read/write (CD-R/W), DVD, and a USB drive.

A data processing system suitable for storing and/or executing program code will include at least one processor (e.g., computer processor 28) coupled directly or indirectly to memory elements through a system bus. The memory elements can include local memory employed during actual execution of the program code, bulk storage, and cache memories which provide temporary storage of at least some program code in order to reduce the number of times code must be retrieved from bulk storage during execution. The system can read the inventive instructions on the program storage devices and follow these instructions to execute the methodology of the embodiments of the disclosure.

Network adapters may be coupled to the processor to enable the processor to become coupled to other processors or remote printers or storage devices through intervening private or public networks. Modems, cable modem and Ethernet cards are just a few of the currently available types of network adapters.

Computer program code for carrying out operations of the present disclosure may be written in any combination of one or more programming languages, including an object-oriented programming language such as Java, Smalltalk, C++ or the like and conventional procedural programming languages, such as the C programming language or similar programming languages.

It will be understood that the algorithms described herein, can be implemented by computer program instructions. These computer program instructions may be provided to a processor of a general-purpose computer, special purpose computer, or other programmable data processing apparatus to produce a machine, such that the instructions, which execute via the processor of the computer (e.g., computer processor 28) or other programmable data processing apparatus, create means for implementing the functions/acts specified in the algorithms described in the present application. These computer program instructions may also be stored in a computer-readable medium (e.g., a non-transitory computer-readable medium) that can direct a computer or other programmable data processing apparatus to function in a particular manner, such that the instructions stored in the computer-readable medium produce an article of manufacture including instruction means which implement the function/act specified in the algorithms. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide processes for implementing the functions/acts specified in the algorithms described in the present application.

Computer processor 28 is typically a hardware device programmed with computer program instructions to produce a special purpose computer. For example, when programmed to perform the algorithms described with reference to the Figures, computer processor 28 typically acts as a special purpose robotic-system computer processor. Typically, the operations described herein that are performed by computer processor 28 transform the physical state of a memory, which is a real physical article, to have a different magnetic polarity, electrical charge, or the like depending on the technology of the memory that is used. For some applications, operations that are described as being performed by a computer processor are performed by a plurality of computer processors in combination with each other.

It will be appreciated by persons skilled in the art that the present disclosure is not limited to what has been particularly shown and described hereinabove. Rather, the scope of the present disclosure includes variations and modifications, which would occur to persons skilled in the art upon reading the foregoing description.

## Claims

1. Apparatus, comprising:
an imaging device (22) configured to image an eye of a patient (42);
a display (24) configured to display a mirror image (50M) of the eye to an operator (40) who has a preference for operating on an opposite eye of the patient;
one or more control components (70) configured to be operated by the operator;
one or more robotic units (20) configured to hold one or more tools (21) and to perform an ophthalmic procedure on the eye using the tools, each of the one or more robotic units corresponding to a respective control component and mirroring a position of the corresponding control component; and
at least one computer processor (28) configured to process inputs into each of the control components such as to generate mirrored outputs at the corresponding robotic unit, such that the operator can perform the procedure on the eye as if the operator were performing the procedure on the opposite eye.

2. The apparatus according to claim 1, wherein the preference for operating on the opposite eye is due to a positioning of a dominant hand that would be required to operate on the eye with the dominant hand, relative to another positioning of the dominant hand that would be possible when operating on the opposite eye with the dominant hand.

3. The apparatus according to claim 1, wherein the procedure includes removal of a natural lens of the eye and replacement of the natural lens with an intraocular lens.

4. The apparatus according to any one of claims 1-3, wherein the one or more control components comprise one or more joysticks.

5. The apparatus according to claim 4, wherein the one or more joysticks are configured to hold virtual tools that corresponds to the one or more tools.

6. The apparatus according to claim 5, wherein the computer processor is configured to cause a tip of any one of the tools to move such that it mirrors movement of a tip of a corresponding one of the one or more virtual tools.

7. The apparatus according to any one of claims 1-3, wherein:
the one or more control components comprise a first control component configured to be operated by a left hand of the operator and a second control component configured to be operated by a right hand of the operator; and
the one or more robotic units comprise a first robotic unit configured to hold a first one of the one or more tools, and a second robotic unit being configured to hold a second one of the one or more tools, the first robotic unit corresponding to the first control component and mirroring a position of the first control component, and the second robotic unit corresponding to the second control component and mirroring a position of the second control component.

8. A computer software product comprising a tangible non-transitory computer-readable medium in which program instructions are stored, which instructions, when read by a computer processor (28), cause the processor to:
perform an ophthalmic procedure on an eye of a patient (42) using one or more tools (21) that are held by one or more robotic units (20), by:
driving an imaging device (22) to image the eye,
driving a display (24) to display a mirror image (50M) of the eye to an operator (40) who has a preference for operating on an opposite eye of the patient,
receiving inputs via one or more control components (70) operated by the operator, wherein each of the one or more robotic units corresponds to a respective control component and mirrors a position of the corresponding control component, and
processing the inputs such as to generate mirrored outputs at the corresponding robotic unit, such that the operator can perform the procedure on the eye as if the operator were performing the procedure on the opposite eye.

9. The computer software product according to claim 8, wherein the preference for operating on the opposite eye is due to a positioning of a dominant hand that would be required to operate on the eye with the dominant hand, relative to another positioning of the dominant hand that would be possible when operating on the opposite eye with the dominant hand.

10. The computer software product according to claim 8, wherein the procedure includes removal of a natural lens of the eye and replacement of the natural lens with an intraocular lens.

11. The computer software product according to any one of claims 8-10, wherein the one or more control components include one or more joysticks.

12. The computer software product according to claim 11, wherein the one or more joysticks hold virtual tools that corresponds to the one or more tools.

13. The computer software product according to claim 12, wherein processing the inputs such as to generate mirrored outputs at the corresponding robotic unit includes causing a tip of any one of the tools to move such that it mirrors movement of a tip of a corresponding one of the one or more virtual tools.

14. The computer software product according to any one of claims 8-10, wherein:
the robotic units include a first robotic unit that holds a first one of the one or more tools, and a second robotic unit that holds a second one of the one or more tools;
the control components include a first control component operated by a left hand of the operator and a second control component operated by a right hand of the operator; and
processing the inputs such as to generate mirrored outputs at the corresponding robotic unit includes moving the first robotic unit such as to mirror a position of the first control component, and moving the second robotic unit such as to mirror a position of the second control component.

## Patentansprüche

1. Einrichtung, umfassend:
eine Abbildungsvorrichtung (22), die dazu konfiguriert ist, ein Auge eines Patienten (42) abzubilden;
eine Anzeige (24), die dazu konfiguriert ist, einem Bediener (40), der eine Präferenz dafür aufweist, an einem gegenüberliegenden Auge des Patienten zu arbeiten, ein Spiegelbild (50M) des Auges anzuzeigen;
eine oder mehrere Steuerungskomponenten (70), die dazu konfiguriert sind, durch den Bediener bedient zu werden;
eine oder mehrere Robotereinheiten (20), die dazu konfiguriert sind, ein oder mehrere Werkzeuge (21) zu halten und unter Verwendung der Werkzeuge ein ophthalmisches Verfahren an dem Auge durchzuführen, wobei jede von der einen oder den mehreren Robotereinheiten einer jeweiligen Steuerungskomponente entspricht und eine Position der entsprechenden Steuerungskomponente spiegelt; und
mindestens einen Computerprozessor (28), der dazu konfiguriert ist, Eingaben in jede der Steuerungskomponenten zu verarbeiten, um gespiegelte Ausgaben an der entsprechenden Robotereinheit zu erzeugen, sodass der Bediener das Verfahren an dem Auge durchführen kann, als ob der Bediener das Verfahren an dem gegenüberliegenden Auge durchführen würde.

2. Einrichtung nach Anspruch 1, wobei die Präferenz für das Arbeiten an dem gegenüberliegenden Auge auf eine Positionierung einer dominanten Hand zurückzuführen ist, die erforderlich wäre, um mit der dominanten Hand an dem Auge zu arbeiten, relativ zu einer anderen Positionierung der dominanten Hand, die möglich wäre, wenn mit der dominanten Hand an dem gegenüberliegenden Auge gearbeitet würde.

3. Einrichtung nach Anspruch 1, wobei das Verfahren Entfernen einer natürlichen Linse des Auges und Ersetzen der natürlichen Linse durch eine Intraokularlinse enthält.

4. Einrichtung nach einem der Ansprüche 1-3, wobei die eine oder mehreren Steuerungskomponenten einen oder mehrere Joysticks umfassen.

5. Einrichtung nach Anspruch 4, wobei der eine oder die mehreren Joysticks dazu konfiguriert sind, virtuelle Werkzeuge zu halten, die dem einen oder den mehreren Werkzeugen entsprechen.

6. Einrichtung nach Anspruch 5, wobei der Computerprozessor dazu konfiguriert ist, eine Spitze eines beliebigen der Werkzeuge zu veranlassen, sich derart zu bewegen, dass sie eine Bewegung einer Spitze eines entsprechenden des einen oder der mehreren virtuellen Werkzeuge spiegelt.

7. Einrichtung nach einem der Ansprüche 1-3, wobei:
die eine oder mehreren Steuerungskomponenten eine erste Steuerungskomponente, die dazu konfiguriert ist, durch eine linke Hand des Bedieners bedient zu werden, und eine zweite Steuerungskomponente, die dazu konfiguriert ist, durch eine rechte Hand des Bedieners bedient zu werden, umfassen; und
die eine oder die mehreren Robotereinheiten eine erste Robotereinheit, die dazu konfiguriert ist, ein erstes von dem einen oder den mehreren Werkzeugen zu halten, und eine zweite Robotereinheit umfassen, die dazu konfiguriert ist, ein zweites von dem einen oder den mehreren Werkzeugen zu halten, wobei die erste Robotereinheit der ersten Steuerungskomponente entspricht und eine Position der ersten Steuerungskomponente spiegelt und die zweite Robotereinheit der zweiten Steuerungskomponente entspricht und eine Position der zweiten Steuerungskomponente spiegelt.

8. Computersoftwareprodukt, umfassend ein physisches nichtflüchtiges computerlesbares Medium, auf dem Programmanweisungen gespeichert sind, wobei die Anweisungen, wenn sie durch einen Computerprozessor (28) gelesen werden, den Computerprozessor zu Folgendem veranlassen:
Durchführen eines ophthalmischen Verfahrens an einem Auge eines Patienten (42) unter Verwendung eines oder mehrerer Werkzeuge (21), die durch eine oder mehrere Robotereinheiten (20) gehalten werden, durch:
Ansteuern einer Abbildungsvorrichtung (22), um das Auge abzubilden,
Ansteuern einer Anzeige (24), um einem Bediener (40), der eine Präferenz dafür aufweist, an einem gegenüberliegenden Auge des Patienten zu arbeiten, ein Spiegelbild (50M) des Auges anzuzeigen,
Empfangen von Eingaben über eine oder mehrere durch den Bediener bediente Steuerungskomponenten (70), wobei jede von der einen oder den mehreren Robotereinheiten einer jeweiligen Steuerungskomponente entspricht und eine Position der entsprechenden Steuerungskomponente spiegelt, und
Verarbeiten der Eingaben, um gespiegelte Ausgaben an der entsprechenden Robotereinheit zu erzeugen, sodass der Bediener das Verfahren an dem Auge durchführen kann, als ob der Bediener das Verfahren an dem gegenüberliegenden Auge durchführen würde.

9. Computersoftwareprodukt nach Anspruch 8, wobei die Präferenz für das Arbeiten an dem gegenüberliegenden Auge auf eine Positionierung einer dominanten Hand zurückzuführen ist, die erforderlich wäre, um mit der dominanten Hand an dem Auge zu arbeiten, relativ zu einer anderen Positionierung der dominanten Hand, die möglich wäre, wenn mit der dominanten Hand an dem gegenüberliegenden Auge gearbeitet würde.

10. Computersoftwareprodukt nach Anspruch 8, wobei das Verfahren Entfernen einer natürlichen Linse des Auges und Ersetzen der natürlichen Linse durch eine Intraokularlinse enthält.

11. Computersoftwareprodukt nach einem der Ansprüche 8-10, wobei die eine oder mehreren Steuerungskomponenten einen oder mehrere Joysticks enthalten.

12. Computersoftwareprodukt nach Anspruch 11, wobei der eine oder die mehreren Joysticks virtuelle Werkzeuge halten, die dem einen oder den mehreren Werkzeugen entsprechen.

13. Computersoftwareprodukt nach Anspruch 12, wobei das Verarbeiten der Eingaben, um gespiegelte Ausgaben an der entsprechenden Robotereinheit zu erzeugen, Veranlassen, dass sich eine Spitze eines beliebigen der Werkzeuge derart bewegt, dass sie eine Bewegung einer Spitze eines entsprechenden von dem einen oder den mehreren virtuellen Werkzeuge spiegelt, enthält.

14. Computersoftwareprodukt nach einem der Ansprüche 8-10, wobei:
die Robotereinheiten eine erste Robotereinheit, die ein erstes von dem einen oder den mehreren Werkzeugen hält, und eine zweite Robotereinheit, die ein zweites von dem einen oder den mehreren Werkzeugen hält, enthalten;
die Steuerungskomponenten eine erste Steuerungskomponente, die durch eine linke Hand des Bedieners bedient wird, und eine zweite Steuerungskomponente, die durch eine rechte Hand des Bedieners bedient wird, enthalten; und
das Verarbeiten der Eingaben, um gespiegelte Ausgaben an der entsprechenden Robotereinheit zu erzeugen, Bewegen der ersten Robotereinheit, um eine Position der ersten Steuerungskomponente zu spiegeln, und Bewegen der zweiten Robotereinheit, um eine Position der zweiten Steuerungskomponente zu spiegeln, enthält.

## Revendications

1. Appareil comprenant :
un dispositif d'imagerie (22) configuré pour imager un œil d'un patient (42) ;
un dispositif d'affichage (24) configuré pour afficher une image miroir (50M) de l'œil à un opérateur (40) qui a une préférence pour opérer sur un œil opposé du patient ;
un ou plusieurs composants de commande (70) configurés pour être actionnés par l'opérateur ;
une ou plusieurs unités robotiques (20) configurées pour tenir un ou plusieurs outils (21) et pour effectuer une acte ophtalmique sur l'œil à l'aide des outils, chacune desdites une ou plusieurs unités robotiques correspondant à un composant de commande respectif et reflétant une position du composant de commande correspondant ; et
au moins un processeur informatique (28) configuré pour traiter les entrées dans chacun des composants de commande de manière à générer des sorties en miroir au niveau de l'unité robotique correspondante, de sorte que l'opérateur puisse effectuer l'acte sur l'œil comme si l'opérateur effectuait l'acte sur l'œil opposé.

2. Appareil selon la revendication 1, ladite préférence pour opérer sur l'œil opposé étant due à un positionnement d'une main dominante qui serait nécessaire pour opérer sur l'œil avec la main dominante, par rapport à un autre positionnement de la main dominante qui serait possible lors d'une opération sur l'œil opposé avec la main dominante.

3. Appareil selon la revendication 1, ledit acte comprenant le retrait d'un cristallin naturel de l'œil et le remplacement du cristallin naturel par un cristallin intraoculaire.

4. Appareil selon l'une quelconque des revendications 1 à 3, lesdits un ou plusieurs composants de commande comprenant un ou plusieurs joysticks.

5. Appareil selon la revendication 4, lesdits un ou plusieurs joysticks étant configurés pour tenir des outils virtuels qui correspondent auxdits un ou plusieurs outils.

6. Appareil selon la revendication 5, ledit processeur informatique étant configuré pour provoquer le déplacement d'une pointe de l'un quelconque des outils de sorte qu'il reflète le déplacement d'une pointe de l'un correspondant desdits un ou plusieurs outils virtuels.

7. Appareil selon l'une quelconque des revendications 1 à 3 :
lesdits un ou plusieurs composants de commande comprenant un premier composant de commande configuré pour être actionné par une main gauche de l'opérateur et un second composant de commande configuré pour être actionné par une main droite de l'opérateur ; et
lesdites une ou plusieurs unités robotiques comprenant une première unité robotique configurée pour tenir un premier outil parmi lesdits un ou plusieurs outils, et une seconde unité robotique étant configurée pour tenir un second outil parmi lesdits un ou plusieurs outils, ladite première unité robotique correspondant au premier composant de commande et reflétant une position du premier composant de commande, et ladite seconde unité robotique correspondant au second composant de commande et reflétant une position du second composant de commande.

8. Produit logiciel informatique comprenant un support tangible non transitoire lisible par ordinateur dans lequel des instructions de programme sont stockées, lesquelles instructions, lorsqu'elles sont lues par un processeur informatique (28), amènent le processeur à :
effectuer une acte ophtalmique sur un œil d'un patient (42) à l'aide d'un ou plusieurs outils (21) qui sont tenus par une ou plusieurs unités robotiques (20), en :
pilotant un dispositif d'imagerie (22) pour imager l'œil,
pilotant un dispositif d'affichage (24) pour afficher une image miroir (50M) de l'œil à un opérateur (40) qui a une préférence pour opérer sur un œil opposé du patient,
recevant des entrées par le biais d'un ou plusieurs composants de commande (70) actionnés par l'opérateur, chacune desdites une ou plusieurs unités robotiques correspondant à un composant de commande respectif et reflétant une position du composant de commande correspondant, et
traitant les entrées de manière à générer des sorties en miroir au niveau de l'unité robotique correspondante, de sorte que l'opérateur puisse effectuer l'acte sur l'œil comme si l'opérateur effectuait l'acte sur l'œil opposé.

9. Produit logiciel informatique selon la revendication 8, ladite préférence pour opérer sur l'œil opposé étant due à un positionnement d'une main dominante qui serait nécessaire pour opérer sur l'œil avec la main dominante, par rapport à un autre positionnement de la main dominante qui serait possible lors d'une opération sur l'œil opposé avec la main dominante.

10. Produit logiciel informatique selon la revendication 8, ledit acte comprenant le retrait d'un cristallin naturel de l'œil et le remplacement du cristallin naturel par un cristallin intraoculaire.

11. Produit logiciel informatique selon l'une quelconque des revendications 8 à 10, lesdits un ou plusieurs composants de commande comprenant un ou plusieurs joysticks.

12. Produit logiciel informatique selon la revendication 11, lesdits un ou plusieurs joysticks tenant des outils virtuels qui correspondent auxdits un ou plusieurs outils.

13. Produit logiciel informatique selon la revendication 12, ledit traitement des entrées de manière à générer des sorties en miroir au niveau de l'unité robotique correspondante comprenant la provocation du déplacement d'une pointe de l'un quelconque des outils de sorte qu'il reflète le déplacement d'une pointe de l'un correspondant desdits un ou plusieurs outils virtuels.

14. Produit logiciel informatique selon l'une quelconque des revendications 8 à 10 :
lesdites unités robotiques comprenant une première unité robotique qui tient un premier outil parmi lesdits un ou plusieurs outils, et une seconde unité robotique qui tient un second outil parmi lesdits un ou plusieurs outils ;
lesdits composants de commande comprenant un premier composant de commande actionné par une main gauche de l'opérateur et un second composant de commande actionné par une main droite de l'opérateur ; et
ledit traitement des entrées de manière à générer des sorties en miroir au niveau de l'unité robotique correspondante comprenant le déplacement de la première unité robotique de manière à refléter une position du premier composant de commande, et le déplacement de la seconde unité robotique de manière à refléter une position du second composant de commande.
